# EUROPEAN PATENT SPECIFICATION

(11) **EP 3 102 941 B1**
(45) Date of publication and mention of the grant of the patent: **03.10.2018**
(21) Application number: 15746070.0
(22) Date of filing: 02.02.2015
(51) Int. Cl.: G01N 33/543

(54) **METHOD FOR THE CHARACTERIZATION OF MULTISPECIFIC SPECIES**
VERFAHREN ZUR CHARAKTERISIERUNG EINER MULTISPEZIFISCHEN SPEZIES
PROCÉDÉ POUR LA CARACTÉRISATION D'ESPÈCES PLURISPÉCIFIQUES

(30) Priority: 07.02.2014 SE 1400061
(43) Date of publication of application: 14.12.2016
(73) Proprietor: Ridgeview Diagnostics AB, 751 83 UPPSALA (SE)
(72) Inventor: ANDERSSON, Karl, 740 20 Vänge (SE)
(74) Representative: Brann AB
(86) International application number: PCT/SE2015/050110
(87) International publication number: WO 2015/119560

(56) References cited:
- WO-A1-2010/033069
- WO-A1-2013/180635
- James P Landry ET AL: "High Throughput, Label-free Screening Small Molecule Compound Libraries for Protein-Ligands using Combination of Small Molecule Microarrays and a Special Ellipsometry-based Optical Scanner", , 1 December 2011 (2011-12-01), XP055384181, Retrieved from the Internet: URL:https://www.ncbi.nlm.nih.gov/pmc/artic les/PMC3271728/pdf/nihms350115.pdf [retrieved on 2017-06-22]
- HANNA BJÏ RKELUND: "Resolving the EGF-EGFR interaction characteristics through a multiple-temperature, multiple-inhibitor, real-time interaction analysis approach", MOLECULAR AND CLINICAL ONCOLOGY, 30 October 2012 (2012-10-30), XP55295469, GR ISSN: 2049-9450, DOI: 10.3892/mco.2012.37
- ALTSCHUH D ET AL.: 'Deciphering complex protein interaction kinetics using Interaction Map' BIOCHEM BIOPHYS RES COMM vol. 428, 2012, pages 74 - 79, XP055175490
- EKERLJUNG L ET AL.: 'Generation and Evaluation of Bispecific Affibody Molecules for Simultanous Targeting of EGFR and HER2' BIOCONJUGATE CHEMISTRY vol. 23, 2012, pages 1802 - 1811, XP055218494
- GARRIDO G ET AL.: 'Bivalent binding by intermediate affinity of nimotuzumab' CANCER BIOLOGY&THERAPY vol. 11, 2011, pages 373 - 382, XP055197571
- BJÖRKELUND H ET AL.: 'Gefitinib Induces Epidermal Growth Factor Receptor Dimer Which Alters the Interaction Characteristics with 1251-EGF' PLOS ONE vol. 6, 2011, page E24739, XP055016196

## Description

### BACKGROUND OF THE INVENTION

### Field of the Invention

The present invention relates to the determination of characteristic parameters for molecular interactions, and more particularly to a method for determining characteristic parameters for the interaction between one or more species immobilized to a solid support surface and binding partners to a multispecific species in solution. The invention also relates to an analytical system, a computer program product and a computer system for performing the method.

### Description of the Related Art

Analytical sensor systems that can monitor interactions between species, such as molecules or biomolecules, in real time are gaining increasing interest. These sensor systems, sometimes referred to as interaction analysis sensor systems or biospecific interaction analysis sensor systems, can be based on different detection principles. Biophysical interaction analysis systems, i.e. systems for the analysis of purified molecules, are often based on optical detectors capable of quantifying the interaction in real time without the need to label the interacting molecules. Such biophysical sensor systems have been used in the study of a variety of biomolecules, including proteins, nucleic acids, lipids and carbohydrates. In these systems, a solid support, often called a sensor surface, having one of the species immobilized thereto is contacted with a solution containing the other species, either by providing a flow of the solution past the sensor surface, or in a cuvette or the like, and binding interactions at the surface are detected. In cell-based interaction analysis systems it is common to have the species in solution labeled with a reporting moiety, such as a radioactive label or a fluorescent label, even though label free cell based interaction analysis systems based on label free detection through optical or acoustic sensors have been described. The cell based assay also typically relies on a solid support onto which cells expressing a species of interest are attached. The cells on the solid support are contacted with a solution containing the other species, either by providing a flow of the solution past the sensor surface, or in a cuvette or the like, and binding interactions at the surface are detected.

The most common use of species in solution is of monospecific nature. This means that the species in solution is intended to bind to one structure so as to effectuate a function. One example is an enzyme inhibitor, where the function of the enzyme inhibitor is to bind to a particular enzyme in a manner that stop the enzyme function. Such a situation is commonly referred to as a one-to-one interaction, i.e. in this particular case one enzyme inhibitor is binding to one enzyme molecule in one way to effectuate the function. The analysis of monospecific molecular interactions is well described in the literature since more than 20 years, and a representative review publication is "Survey of the 2009 commercial optical biosensor literature." By Rich RL and Myszka DG as published in J Mol Recognit. 2011 Nov-Dec; 24(6):892-914.

In biology, multispecific species are however common. One illustrative example is found in the immune system, where an antibody is designed to bind to a pathogen in one end of the molecule and designed to interact with the cellular machinery of the immune system (T-cells, NK cells, and others). In simplified terms, the job of the antibody is to link a pathogen to the cells that kill pathogens. This means that the antibody has two simultaneous functions that are mediated by molecular interactions and hence it is a multispecific species.

In the pharmaceutical industry, it is becoming increasingly common to evaluate molecules that are multispecific with respect to the binding to the pathogen or tumor for the suitability as therapeutic entities. One rationale for such an approach is that if two structures that are abundantly present on the pathogen or the tumor are targeted at the same time, the multispecific molecule will discriminate the pathogen or tumor from normal tissue. This is under the assumption that normal tissue does not commonly express the combination of two targets. With such a multispecific design, it will become difficult to characterize the interaction of the multispecific species to the combination of species on the pathogen or tumor, because the interaction as such will be a combination of multispecific species binding to one or both of the target structures.

There are examples of bispecific antibodies, where the originally symmetric antibody molecule has been engineered to present two different antigen-recognizing domains, so as to bind to two different targets on the same pathogen or tumor (while as still having the capacity to attract the immune system components). Such antibodies have been described previously, for example in the report "A two-in-one antibody against HER3 and EGFR has superior inhibitory activity compared with monospecific antibodies." by Schaefer G and co-authors as published in Cancer Cell. 2011 Oct 18;20(4):472-86. Interaction Map is a multidimensional fingerprinting method for separating signals from different interaction evens that occur in parallel. One description of Interaction Map is found in the report "Deciphering complex protein interaction kinetics using Interaction Map" by Altschuh and co-authors as published in Biochem Biophys Res Commun. 2012 Nov 9;428(1):74-9.

This report describes the analysis of a monospecific reagent, the FAb fragment 57P, interacting with two similar peptides using Interaction Map.

### Summary of the invention

The present invention is based on the discovery that in interaction behavior of multispecific species with their targets can be better understood through use of multidimensional fingerprint analysis. This can result in major improvements in the development and selection of multispecific therapeutic agents.

It is an object of the present invention to provide a sensor-based method for determining how multispecific species interact with the intended targets.

Accordingly, based on the discoveries of the present invention, one aspect of the present invention provides a method for the characterization of a multispecific species being capable of binding at least two different defined targets, comprising the steps of:
a) Providing, for each target, at least one solid support with only one target immobilized
b) Providing at least one solid support with at least two targets immobilized
c) Contacting each solid support having only one target immobilized with a liquid containing a predefined concentration of said multispecific species and detecting in a time resolved manner the progress of the interaction of said multispecific species with the target on said solid support, so as to create at least one single-target binding curve for each target
d) Contacting each solid support having at least two targets immobilized with a liquid containing a predefined concentration of said multispecific species and detecting in a time resolved manner the progress of the interaction of said multispecific species with the targets on said solid support, so as to create at least one multi-target binding curve for each combination of targets.
e) Processing each binding curve in a processor to produce a multidimensional fingerprint, said multidimensional fingerprint being a representation of the binding curve being processed
f) In each of the multidimensional fingerprints of single-target binding curves, identifying the single dominant feature as the characteristic value for the isolated interaction of said multispecific species to the only target immobilized on the solid support
g) In the multidimensional fingerprint of a multi-target binding curve, comparing the features of the multi-target interaction map to the characteristic values of the single-target interaction maps for the targets present in said multi-target interaction map and calculating the greatest improvement from any of the characteristic values to the closest feature(s) in the multi-target interaction map as the change in binding characteristics due to multispecificity. The calculation of the greatest improvement is conducted as one of
   a. Improvement in terms of apparent binding affinity
   b. Improvement in terms of reduced dissociation rate
   c. Improvement in terms of increased association rate
   a. Improvement in terms of weight of the dominant peak

The output of the method provides detailed information about how said multispecific species binds to a solid support holding a mix of different targets.

In an embodiment, the steps of contacting a solid support with said multispecific species comprises using two different concentrations of said multispecific species, which increases the information content.

In another embodiment, the multidimensional fingerprint is the Interaction Map method.

In still another embodiment, the characteristic value for the isolated interaction is one of the following:
a) The position of the dominant peak
b) The affinity derived from the position of the dominant peak
c) The weight of the dominant peak
d) The association rate value of the dominant peak
e) The dissociation rate value of the dominant peak
f) The width of the dominant peak

In yet another aspect of the invention, the binding curves are detected in an instrument based on the detection of refractive index near the surface of a solid support.

In still another aspect of the invention, the binding curves are detected in an instrument having one of the following detection principles (a) Surface Plasmon Resonance, (b) Quartz Crystal Microbalance, (c) Bio-Layer Interferometry or (d) Surface Acoustic Wave.

In yet another embodiment, the multispecific species is labeled with either a fluorescent label or a radioactive label, and wherein said binding curves are detected using a method which relies on the temporary reduction of liquid during quantification of bound multispecific species.

In still another embodiment, each of the targets is a protein with molecular weight exceeding 5000 Da and wherein any of said different targets share less than 98% of the amino acid sequence with any other of said different targets.

In still another aspect of the invention, a method for the characterization of a biological sample by use of a multispecific species, said multispecific species being designed to bind at least two different defined targets is disclosed. This method comprises the steps of
a) Providing a biological sample
b) Contacting said biological sample with a liquid containing a predefined concentration of said multispecific species and detecting in a time resolved manner the progress of the interaction of said multispecific species with the biological sample, so as to create at least binding curve for said biological sample
c) Processing each binding curve in a processor to produce a multidimensional fingerprint, said multidimensional fingerprint being a representation of the binding curve being processed
d) Comparing selected features of said multidimensional fingerprint to predefined values to determine if said biological sample express said at least two different targets so as to classify said biological sample as indicative of disease

The predefined values of features of said multidimensional fingerprint for said multispecific species is determined by comparing multidimensional fingerprints obtained for biological samples known to express only one of said at least two targets to multidimensional fingerprints obtained for biological samples known to express said at least two different targets.

### BRIEF DESCRIPTION OF THE DRAWINGS

FIG. 1 Describes a time-resolved measurement of a molecular interaction and an accompanying multidimensional fingerprint derived from said measurement result.
FIG. 2 Describes a time-resolved measurement and accompanying multidimensional fingerprints of a molecular interaction between a multispecific molecule (molecule I) and three different solid supports.
FIG. 3 Describes a time-resolved measurement and accompanying multidimensional fingerprints of a molecular interaction between a multispecific molecule (molecule III) and three different solid supports.

### DETAILED DESCRIPTION OF THE INVENTION

Unless defined otherwise, all technical and scientific terms used herein have the same meaning as commonly understood by a person skilled in the art related to this invention. Also, the singular forms "a", "an", and "the" are meant to include plural reference unless it is stated otherwise.

For the purpose of the present invention and for clarity, the following definitions are made.

A "species" is defined as a molecule, potentially capable to interact in a specific manner with other species. Possible species include, but are not limited to, protein molecules (i.e. amino acid polymers with molecular weight exceeding approximately 5000 Da), peptide molecules (i.e. amino acid polymers with molecular weight exceeding approximately 500 Da while not being a protein), oligonucleotides (i.e. nucleic acid polymers exceeding with molecular weight exceeding approximately 500 Da), and chemical compounds (including but not limited to synthetic molecules and endogenous chemical compounds such as dopamine) of molecular weight exceeding 250 Da.

The term "multispecific species" refers to a species which has two distinct binding sites with different specificities. One non-limiting example of a multispecific species is a molecule made of two independent proteins with different binding specificities being held together through a linker molecule. Another non-limiting example of a multispecific species is an engineered antibody wherein the typically symmetric form of antibodies has been altered to introduce a different CDR3 loop in one of the two arms of the antibody. Such antibodies are commonly denoted bispecific antibodies. The two distinct binding sites located on a multispecific species are typically non-overlapping, and may be located 0.1 nm apart from each other, or 1 nm apart, or even 10 nm apart. Multispecific species may be designed by man or may be naturally occurring.

A "target" is defined as a species with a function that a drug is intended to alter. The structure is commonly a protein molecule, but can be other biomolecules as well including RNA molecules, DNA molecules, carbohydrate molecules and the similar. To mention one non-limiting example, a target may be a growth hormone receptor, such as the Epidermal Growth Factor Receptor (EGFR) which is a target relevant for various cancer diseases.

The term "different targets" indicate that the two targets being different in any of the following aspects: (a) share less than 99%, or 98%, or 95% of amino acid sequence in case the targets are proteins of molecular weight exceeding 5000 Da, (b) share less than 99%, or 98%, or 95% nucleic acid sequence in cases targets are nucleic acids of molecular weight exceeding 5000 Da, or (c) are differently modified by the hosting cell in terms of different glycosylation pattern, ubiquitination pattern, and/or methylation pattern.

A "solid support" denotes a solid structure for use in an analytical procedure wherein the interaction between species is detected. Examples of a solid support include, but are not limited to, gold-coated glass slides (or transparent plastic slides) for Surface Plasmon Resonance (SPR) measurements, Quartz crystals for Quartz Crystal Microbalance measurements, thin layer coated glass or plastic slides for interferometry measurements, optical fibers coated with gold or other coatings for SPR or interferometry measurements, and plastic Petri dishes for LigandTracer measurements.

The term "characteristic value" refers to a value derived from one or more features of a multidimensional representation of measured data for one compound. One example of a characteristic value is the area under the measured curve of the compound interacting with a biological structure. Another example of a characteristic value is the peak position of the dominant peak in an interaction map calculated for a compound interacting with a biological structure. The characteristic value is typically a scalar numerical value.

The term "primitive curves" denotes a plurality of curves, which in linear combination may reproduce a majority of the expected possible measured curves. For example, in the case of molecular interactions a suitable set of primitive curves includes (but is not limited to) a collection of curves where each curve represents a monovalent interaction with unique pair of association rate and dissociation rate values. Given a sufficient number of such primitive curves, it is possible to reproduce the data obtained from the measurement of a molecular interaction with a linear combination of said primitive curves. The concept of primitive curves is equivalent to the concept of a base vector system, wherein any monotonous curve can be expressed as a linear combination of a base vectors, provided that the base vector system is complete.

The term "multidimensional fingerprint" refers to a non-scalar numerical representation of one or more time-resolved measurements. A multidimensional fingerprint may be constructed through the use of primitive curves, wherein the time-resolved measurement is reconstructed as a linear combination of said primitive curves. The coefficients in the linear combination, sometimes referred to as weights, can be utilized as the multidimensional fingerprint. A multidimensional fingerprint comprises preferably more than 10 different numerical values, even more preferably more than 100 different numerical values.

The term "region" in the context of defining a region in a multidimensional fingerprint is a delimited subspace in said fingerprint, typically extending less than two orders of magnitude in any given direction. As a non-limiting example, in the case where the multidimensional fingerprint is an Interaction Map a possible region could be defined by ranges like (4<log10(ka)<5.5 and-5<log10(kd)<-3), or it could be a circular region having a center position (e.g. log10(ka)=4.1; log10(kd)=-3.4) and a radius (e.g. R = 0.6). In some cases even smaller regions are desirable, such as extending 1 order of magnitude in any direction, or even 0.5 orders of magnitude in any direction.

The term "interaction" in the context of a compound interaction with a biological object is defined as the compound having direct or indirect impact on a target receptor. A direct interaction includes, but is not limited to, the compound binding to the target receptor. An indirect interaction includes, but is not limited to, compound affecting the biological object in a manner that in turn causes alterations of the target receptor. A few possible alterations include compound binding to a cofactor necessary for the target receptor to function, compound inhibiting the production of target receptor which effectively reduces the number of target receptors, compounds binding to a dimerization partner in a manner that affect dimerization balance for the target receptor, to mention some non-limiting examples.

A "biological sample" is defined as a small portion of tissue excised from an individual or an animal, including but not limited to portions of body fluid (e.g. blood sample, saliva sample, spinal fluid sample and similar), and solid tissue samples (e.g. biopsies, excess material from surgery, skin grafts and similar).

"Tissue sample" is defined as a biological sample comprising a solid biological object and include, but is not limited to, excess material from surgery, biopsies, embedded tissue samples and sections thereof. A tissue sample is thinner than 1 mm, and is typically thinner than 0.1 mm. The tissue sample further has an area less than 100 cm2, and typically said area is greater than 1 mm2 and less than 10 cm2. The tissue 25 sample under analysis is attached to a solid support and the predefined probes designed to interact with structures on the tissue sample are present in a liquid that is in contact with said solid support. The tissue sample under investigation can be prepared using different methods. One common method is to embed the tissue sample in paraffin according to common IHC protocols, followed by slicing thin 30 sections for attachment to the solid support and analysis using the method of this invention. It is further possible to use fresh-frozen tissue, sliced into thin sections, attached onto the solid support and analyzed using the method of this invention. Tissue sample can also be blood samples analyzed either as blood smears fixed in different ways or as living cells by flow cytometry.

One aspect of the present invention can be described as a method for assessing the binding characteristics of a first species in solution capable of binding to at least two other different species (denoted different targets), said method comprising the following steps:
1. Providing, for each target, at least one solid support with only one target immobilized
2. Providing at least one solid support with at least two different targets immobilized
3. Contacting each solid support having only one target immobilized with a liquid containing a predefined concentration of said multispecific species and detecting in a time resolved manner the progress of the interaction of said species with the target on said solid support, so as to create at least one single-target binding curve for each target
4. Contacting each solid support having at least two different targets immobilized with a liquid containing a predefined concentration of said multispecific species and detecting in a time resolved manner the progress of the interaction of said species with the targets on said solid support, so as to create at least one multi-target binding curve for each combination of targets.
5. Processing each binding curve in a processor to produce a multidimensional fingerprint, said multidimensional fingerprint being a representation of the binding curve being processed
6. In each of the multidimensional fingerprints of single-target binding curves, identifying the single dominant feature as the characteristic value for the isolated interaction of said multispecific species to the only target immobilized on the solid support
7. In the multidimensional fingerprint of a multi-target binding curve, comparing the features of the multi-target interaction map to the characteristic values of the single-target interaction maps for the targets present in said multi-target interaction map and calculating the greatest improvement, for example in terms of apparent binding affinity, of any of the characteristic values as the change in binding characteristics due to multispecificity. Alternative calculations of greatest improvement includes improvement in terms of reduced dissociation rate, improvement in terms of increased association rate and improvement in terms of weight of the dominant peak.

In other words, this means that the method is capable of resolving the cooperative effect of the multispecific species being bound to two or more targets from the situation of the multispecific species binding only to one of the targets. The case where the multispecific species is bound to the solid support through simultaneous binding to the two or more different targets will in most cases increase the apparent binding strength of the multispecific species to the mix of targets. This is typically a wanted feature in the pharmaceutical industry.

The step of attaching species to a solid support differs depending on the type of analytical instrument. In the case of analytical instruments for the biophysical characterization of molecular interactions, including but not limited to surface Plasmon resonance (SPR), Quartz crystal microbalance (QCM), and bio-layer interferometry (BLI), one common method to attach or immobilize a target is through chemical activation of a surface, making it reactive to primary amine groups. For example, in the SPR devices denoted Biacore provided by GE Healthcare, there is a sensor chip which in the most common form is a gold-coated glass slide to which carboxymethylated dextran moieties are attached. The carboxymethylated dextran can be activated using EDC and NHS which results in reactive esters that will covalently anchor primary amines, as described in the report "Immobilization of proteins to a carboxymethyldextran-modified gold surface for biospecific interaction analysis in surface plasmon resonance sensors." by Johnsson B and co-authors as published in Anal Biochem. 1991 Nov 1;198(2):268-77. All proteins have a primary amine in the N-terminal of the polypeptide chain, but can have multiple primary amines since the amino acid lysine presents a primary amine in its side chain. Alternative procedures for attaching or immobilizing species to a solid support for use in a biophysical analytical instrument include, but are not limited to, adsorption of the target to a hydrophobic surface, attaching a tag-recognizing protein to the solid support and capturing a tagged target (for example using streptavidin coating of the solid support and capturing biotinylated target), using other chemistries for attaching a protein to the solid support (including but not limited to aldehyde coupling and thiol coupling, to mention a few non limiting examples. In other analytical instrument, in particular ones dedicated for cell-based analysis, the target is typically expressed on the cell surface and the object of the attachment is to anchor cells to the solid support. In some cases, cells adhere spontaneously to surfaces. In other cases, specific coatings may be required for cells to attach, such as collagen coating or gelatin coating.

The steps of contacting each solid support with at least one concentration of multispecific species can be achieved in different manners. The procedure is commonly denoted "kinetic analysis" and typically comprises contacting a solid support with multiple (2-10) different concentrations of species. Sometimes, the different concentrations are contacted one at a time, intersected with a regeneration procedure which releases bound species from the immobilized target, a procedure often called "multi cycle kinetics". Another option is to contact the target with a sequence of increasing concentrations, either with interleaving dissociation phases (where the concentration of species is zero) or a titration of consecutively increasing concentrations of species. It has been described that a solid support can be contacted with a gradient of species, which in one way is a continuous titration. All these and all similar procedures for generating time resolved interaction data from species binding to an attached target are capable of producing data suitable for the present invention. Suitable analytical instruments for the purpose of kinetic analysis of purified species include, but are not limited to Surface Plasmon Resonance (SPR) instruments, Quartz crystal microbalance (QCM) instruments, Surface Acoustic Wave (SAW) instruments, and bio-layer interferometry (BLI) instruments. Suitable analytical instruments for the purpose of kinetic analysis where the targets are expressed on cells include, but are not limited to SPR instruments, QCM instruments, SAW instruments, and instruments relying on the temporary reduction of liquid during detection of a species labeled with either a fluorescence or a radioactive label, such as LigandTracer.

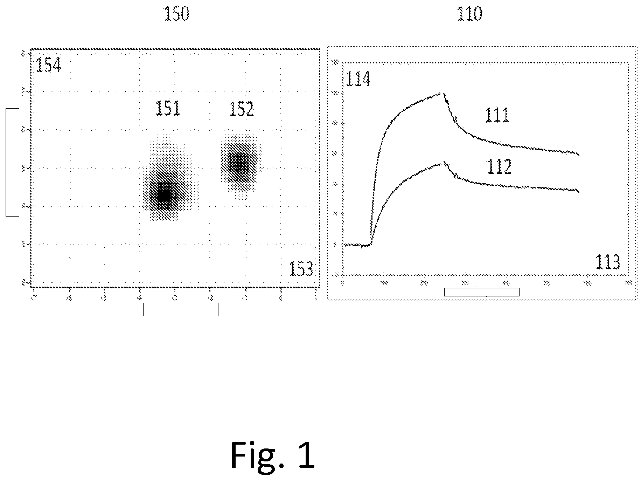

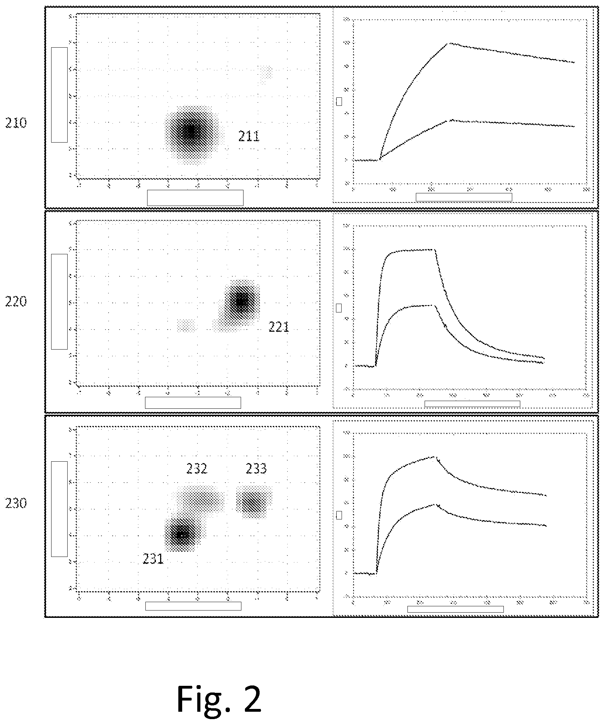

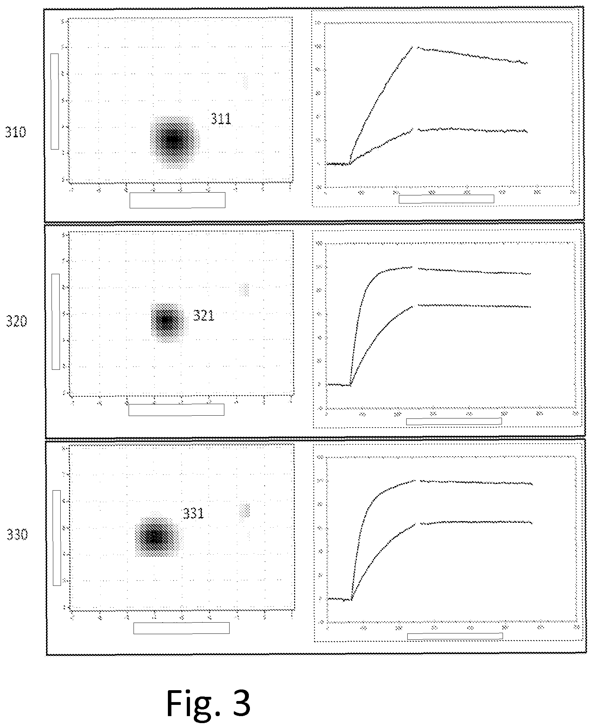

A non-limiting illustration of how a time-resolved interaction measurement can be analyzed using a multidimensional fingerprint is shown in Figure 1. Results from a time resolved interaction measurement (110) comprising two different concentrations (curves 111 and 112) are plotted with time in unit seconds (113) on the x-axis and arbitrary signal units (114) on the y-axis. The arbitrary signal units are proportional to the amount of bound species to the immobilized targets on the solid support. The binding curves (111, 112) can be subjected to analysis using a multidimensional fingerprint, for example the Interaction Map method. An Interaction Map (150) reports the number of parallel interaction like events in the form of a topographic map. The peaks 151 and 152 represent two different interaction-like events. Each peak has a cumulative weight, i.e. a value of the total contribution of the interaction related to a peak to the total interaction. The cumulative weight is typically calculated as a sum of the individual weights for the primitive curves that constitute the peak as such, and in technical terms that is the same as adding the pixel values for the pixels being part of the peak. The cumulative weight is often referred to as only weight. The unit on the x-axis (153) of the Interaction Map is log10(dissociation rate constant) and the y-axis (154) is log10(association rate constant). The structure and the axis units of Figure 1 are applied also in Figure 2 and 3 in this document. A similar case where time-resolved interaction measurement results using purified molecules was analyzed using a multidimensional fingerprint has been disclosed in the report "[(99m)Tc(CO)(3)] (+)-(HE) (3)-Z (IGF1R:4551), a new Affibody conjugate for visualization of insulin-like growth factor-1 receptor expression in malignant tumours" by Orlova and co-authors as published in Eur J Nucl Med Mol Imaging. 2013 Feb;40(3):439-49. Another similar case where time-resolved interaction measurement results using a cell based assay was analyzed using a multidimensional fingerprint has been disclosed in the report "Gefitinib induces epidermal growth factor receptor dimers which alters the interaction characteristics with 1251-EGF" by Bjorkelund and co-authors as published in PLoS One. 2011;6(9): e24739.

The step of processing each binding curve in a processor to produce a multidimensional fingerprint has been described previously in the patent application US2011195434. In brief, producing a multidimensional fingerprint comprises a computer program product directly loadable into the internal memory of a digital computer, wherein the computer program product comprises software code means for performing calculations necessary to express a binding curve as a sum of a predefined set of primitive binding curves, each such primitive curve being multiplied with a weight to adjust the amplitudes of the different primitive curves in the sum that represents the measured binding curve. Thus, each measured binding curve can be represented by a plurality of weights, each weight being associated to a primitive curve. Such a collection of weights is referred to as a vector of weights. Different measured binding curves will be expressed as different vectors of weights. In some cases, the vector of weights can in turn be presented as a topographic map, where the surface of triplets (typically [association rate, dissociation rate, weight]) is plotted as a grayscale plot (Illustrated in Figure 1, 150). Each "peak" (151, 1522) in this plot means that the corresponding association and dissociation rate values have elevated weights, which means that the binding curve (111, 112) is partly composed of an interaction of the corresponding association and dissociation rate values, which in turn means that the multispecific species is interacting with the target with the corresponding association and dissociation rate values. Each species - target interaction will result in at least one such "peak", and the locations and relative heights of the "peak" for a given target or mix of targets will represent complete interaction profile of the multispecific molecule. In some cases a "peak" is discussed in terms of having a weight, which refers to the contribution of said peak to the total amount of binding.

The step of identifying the single dominant feature as the characteristic value for the isolated interaction of said multispecific species typically comprises identifying one or more peaks in an Interaction Map and extracting a suitable value from said identified peak or peaks. Common characteristic values include, but are not limited to the position of the peak, the width of a peak, the affinity represented by the position of the peak, the weight of a peak, or any similar characteristic of the peak. When comparing two peaks, it is often suitable to compare a characteristic value for the two peaks, for example comparing the affinities represented by two different peaks, or comparing the weights of two different peaks.

One of the unique features of the present invention is the ability to distinguish order of events in the interaction of multispecific species. A multispecific species harbors at least two interacting elements, and these interactions may be very different in terms of temporal profile. This means that the comparison of multidimensional fingerprints of solid supports having a single target attached and solid supports having a plurality of targets attached may reveal in which order the multispecific species binds to the solid support. For example, if a multispecific species harbors two interactions of which one is fast and the other is slow, a multidimensional fingerprint can be capable of separating the events where (a) the multispecific molecule first interacts with the target resulting in a fast interaction followed by anchoring the through the slow interaction, and (b) the multispecific molecule first interacts with the target resulting in a slow interaction followed by further strengthening the binding through the fast interaction. In cases where the two interactions are sufficiently different, these two events may have different overall binding properties of which one could be effective from a therapeutic perspective and the other not. Current methods are not capable of distinguishing orders of events and hence there is a risk that a multispecific species wherein one particular order of interaction events results in an overall binding acceptable in therapeutic development are discarded in the development process.

There might be different purposes for using a multispecific species for therapeutic purposes. One non-limiting example is the use of multiple specificities to increase attachment strength of the multispecific species to a cell (e.g. a tumor cell). The increased strength is achieved through the multispecific species attaching to multiple, different targets present on the cell which creates an avidity effect. The size of the multifunctional Ab varies with construction principle and the small Diabody, described in EP0672142 construct is two scFv antibody fragments folded together into one small multifunctional species. This avidity effect has also been described for very small protein molecules in the report "Generation and evaluation of bispecific affibody molecules for simultaneous targeting of EGFR and HER2." by Ekerljung and co-authors as published in Bioconjug Chem. 2012 Sep 19;23(9):1802-11. Another non-limiting example is to use the multispecific species to link cells to each other. In this case, the multispecific species bind with one interaction with a defined target on the first cell type, and anchors the second cell type through another specific interaction to a different target present only on the second cell type. One example of linking cells is Catumaxomab (trade name Removab) which interacts with the EpCAM antigen on a tumor cell and with T-cells carrying CD3, all in combination with the Fc interaction to other cells such as macrophage, dendritic cells and the similar. Another such example was presented in the report "Engineering and characterization of a bispecific HER2×EGFR-binding affibody molecule" by Friedman and co-authors as published in Biotechnol. Appl. Biochem. (2009) 54, 121-131. Still other more technical uses of bifunctional molecules has been described for functionalizing sensor surfaces for diagnostic or other analytical purposes as described in WO9005305.

The findings of the present invention can influence how multispecific species are selected in a drug discovery process due to the relationship between tissue penetration and apparent affinity. Any drug, and in particular protein therapeutics, need to be optimized with respect to multiple properties. One non-limiting example of such a property is the ability of the intended drug to interact with its target. Another non-limiting example of such a property is the ability of the drug to penetrate tissue. Tissue penetration is of essence in many types of diseases, including cancer. Several properties of a molecule have direct impact on tissue penetration, including but not limited to:
- Solubility meaning the balance between hydrophilic and hydrophobic properties of the multispecific species and it is well established that molecules such as polyethylene glycol (PEG) modified protein change these properties.
- Molecular size where it is now well established that smaller proteins like (for example Affibody® molecules) penetrate tissue easier than larger molecules such as full sized antibodies like IgG.
- Affinity also has an impact on penetration depth in tissue where the higher affinity (stronger binding) binders are accumulated to the surface of an exposed tissue.

The impact of affinity and tissue penetration has been discussed in the report "Influence of Affinity and Antigen Internalization on the Uptake and Penetration of Anti-HER2 Antibodies in Solid Tumors." by SI Rudnick and co-authors as published in Cancer Res. 2011 March 15; 71(6): 2250-2259. In brief, the affinity and kinetic properties for a series of antibody scFv fragments against HER2 was analyzed for their tissue penetration properties. To reach deep penetration it was important not have too high affinity. With the delicate balance between time constants and uptake in the cell as well as the penetration depth in tumor tissue it is important to select molecules according to the methods in this invention to increase penetration and retention time based on two or more binding characteristics that can be optimized together. Penetration is taking place when the multispecific species is not bound to the target and can diffuse in the tissue. With the method for characterization of multispecific species it is possible to improve the design multispecific species that are optimized for penetrations as well as for binding and a suitable balance in between. Penetration is possible to design as the time when the multispecific species is not bound to the target giving rise to optimization of penetration. With multiple interaction properties as demonstrated in the examples in this document it is possible to select molecules that are optimized both for binding and penetration. When it comes to multispecific species, another dimension is added to the tissue penetration case: target distribution. In solid tumor cancer disease, the center of a tumor has access to less nutrition than the surface due to the fewer number of blood vessels in a tumor, and this can in theory cause the tumor cells to behave differently within the same tumor. If, for example, there is a multispecific species available capable of binding to target T1 and T2, and it is known that the target T1 is present predominantly on the surface of a solid tumor while as the target T2 is present predominantly in the center of the tumor, the design of a multispecific species capable of reaching the center will be critical to obtain desired results. Firstly, the multispecific species should bind to T1 to accumulate the multispecific species in the tissue. However, the binding to T1 must be weak enough and the multispecific species must be physically small enough for the multispecific species to release and diffuse towards the center. Once in the center, the binding to T2 can preferably be strong. With such a design a multispecific species would likely be superior to a monospecific species with an intended interaction to T2 alone.

A multispecific species may interact with different targets for different purposes. One non-limiting possibility is to use a multispecific species capable of binding to multiple different cell surface targets so as to increase the binding strength (or apparent affinity) to cells that expresses all the different targets. This means that the binding strength to cells expressing all the different targets will be strong, while as the binding strength to cells expressing one or a few of the different targets will be weaker. Hence, the multispecificity can increase both binding strength and specificity. Another non-limiting possibility is to use the multiple binding options of a multispecific species for different purposes. For example, one of the targets for the multispecific species can have the function of binding the multispecific species to a cell, and another target for the multispecific species may trigger an internalization event upon binding. Yet another non-limiting example is to use one target interaction to anchor the multispecific species to a cell and another target interaction to attract the immune system components, such as natural killer cells. Still another non-limiting example is to use one target interaction to anchor the multispecific species to a cell followed by internalization and another target interaction which induces toxic effects through binding to an intracellular protein of crucial function for cell survival, as discussed in the report "Site-specific antibody drug conjugates for cancer therapy" by S Panowski and co-authors as published in mAbs 6:1, 34-45; January/February 2014. In all these examples, it is relevant to investigate the interaction of the multispecific species with all intended targets at the same time, wherein the present invention can be of use for interpreting the results. These different situations are all part of the mechanism of action description, which is of great importance for new therapeutic agents of any kind.

The present invention can also be used for the characterization of a target surface or a biological sample by use of a multispecific species as probe. When a multispecific species has been characterized in terms of binding characteristics to solid supports holding one or multiple targets to which the multispecific species is capable to bind to, a different solid support with unknown properties can be characterized using the multispecific species/probe. This can for example be of interest in cancer diagnostics, because different receptors are sometimes forming dimers, and particular dimer formation may be indicative of disease. One example of development of molecules suitable for dimer detection in cancer has been disclosed in the report "Development of bispecific molecules for the in situ detection of protein-protein interactions and protein phosphorylation." by van Dieck as published in Chem Biol. 2014 Mar 20;21(3):357-68. Doi: 10.1016/j.chembiol.2013.12.018. When applying the present invention as a method for the characterization of a biological sample, a multispecific species with known binding characteristics to (a) single targets and (b) multiple targets is brought in contact with a biological sample, and the progress of the interaction is recorded in a time-resolved manner. The resulting binding curve is subjected to multidimensional fingerprint analysis, preferably using the Interaction Map method, and the output is compared to the known multidimensional fingerprint features of the multispecific species binding to either single targets or multiple targets. Hence, the multispecific species used as probe has to be thoroughly characterized before being used as a reference point or a calibration for classifying unknown biological samples. If the features of multidimensional fingerprint obtained in the biological sample is similar to the known features of the multispecific species binding to multiple targets, then the biological sample is classified as having multiple targets expressed, which in turn can be related to presence of disease or aggressiveness of disease. One situation where such an analysis would be favorable is in the analysis of tissue samples using time-resolved immunohistochemistry, such as described in the report "Evaluation of real-time immunohistochemistry and interaction map as an alternative objective assessment of HER2 expression in human breast cancer tissue", authored by Gedda L and co-authors as published in Appl Immunohistochem Mol Morphol. 2013 Dec;21(6):497-505. (doi: 10.1097/PAI.0b013e318281162d). Another situation where such an analysis would be favorable is in the analysis of cells (e.g. blood cells or circulating tumor cells) using time-resolved FACS (Flow assisted cell sorting), where the multispecific species is allowed to interact with cells in suspension, hence allowing the determination if cells from e.g. a blood sample has multiple target expressed. In this case, the use of a solid support is not required.

### Example 1

All experimental data in this example was originally described in the report "Generation and Evaluation of Bispecific Affibody Molecules for Simultaneous Targeting of EGFR and HER2" by L Ekerljung and co-authors as published in Bioconjugate Chemistry. 2012 Sep 19;23(9):1802-11. In brief, the report describes six bifunctional molecules, all being capable of interacting with the receptors HER2 and EGFR. The bispecific molecules were artificially made through linking one Affibody molecule binding to HER2 to another affibody molecule binding to EGFR in one gene construct. At the time of publishing, there were no adequate tools for analysis of multispecific interactions. This example relates to bispecific molecule I in Table 1 in the report by Ekerljung cited above. The time resolved binding curves for bispecific molecule I binding to (a) EGFR, (b) HER2 and (c) a mix of EGFR+HER2 were subjected to Interaction Map analysis.

Figure 2 shows the time resolved binding curves and the respective Interaction Map results. In more details, when processing the data from bispecific molecule I binding to a solid support where only EGFR was immobilized, the Interaction Map results (210) in a single dominant peak (211) located at position log10(ka) = 3.6 and log10(kd) = -3.2 which represents an affinity of 159 nM, which is also the characteristic value of the EGFR interaction. When processing the data from bispecific molecule I binding to a solid support where only HER2 was immobilized, the Interaction Map results (220) in a single dominant peak (221) located at position log10(ka) = 5.0 and log10(kd) = -1.6 which represents an affinity of 296 nM, which is also the characteristic value of the HER2 interaction. The affinity values extracted using Interaction Map are approximately a factor three different from the values reported in Table 1 in the report by Ekerljung as cited above. This discrepancy is most probably due to the fact that Interaction Map and conventional regression analysis using a 1:1 model have completely different abilities to describe the heterogeneity that is always present also in measurements on purified samples. The differences in results is also comparable to benchmarking studies, such as "Comparative analyses of a small molecule/enzyme interaction by multiple users of Biacore technology." by Cannon and co-authors as published in Anal Biochem. 2004 Jul 1;330(1):98-113.

When processing the data from bispecific molecule I binding to a solid support where a mix of EGFR and HER2 were immobilized, the Interaction Map resulted (230) in a complex pattern. There is a dominant peak (231) in the lower left located at position log10(ka) = 4.0 and log10(kd) = -3.5 which represents an affinity of 27 nM, representing approximately 43% of the weight in the map. There are two additional peaks. The peak in the middle (232) is located at position log10(ka) = 5.2 and log10(kd) = -3.9 which represents an affinity of 6.8 nM, representing approximately 23% of the weight in the map, and which is also the characteristic value of the mixed surface interaction. The peak to the right (233) located at position log10(ka) = 5.2 and log10(kd) = -1.2 which represents an affinity of 416 nM, representing approximately 22% of the weight in the map.

The comparison of the three maps (210, 220, and 230) provides a detailed view of how the bispecific molecule is interacting with a mixed target surface. Peak 233 is located at approximately the same position as peak 221. The most likely explanation for peak 233 is that it describes the bispecific molecule binding to the mixed target solid support with the HER2 moiety, and releases before the EGFR moiety has had time to anchor the bifunctional molecule. As estimated from the peak 221, the bispecific molecule will when binding to HER2 alone only stay in complex a few minutes before it releases. The peak 232 has approximately the same association rate as peak 233 and 221. Since association rate is determined by the first interaction event, it is most likely that 232 describes the event of bispecific molecule first binding to a HER2 target (giving the association rate of the HER2 interaction) followed by the bispecific molecule binding to EGFR to provide the stability of the binding (i.e. the dissociation rate). The interaction represented by 232 is approximately 20 times stronger (which can also be phrased as the apparent affinity of interaction represented by 232 is 20 times higher) than any of the individual interactions. The peak 231 has approximately the same association rate as the peak 211, and peak 231 most likely describes the bispecific molecule first binding to EGFR, followed by stabilization through binding to HER2 with an apparent affinity 5 times stronger than any of the individual interactions. Since the interaction to EGFR alone results in the bispecific molecule residing in complex for a relatively long time (approximately 1 h), there is enough time for the HER2 binding to occur. Hence there are no or very few interactions where bispecific molecule binding to EGFR and releasing before the HER2 binding has stabilized it.

The conclusion of this analysis is that through the use of three different measurements and Interaction Map analysis, it becomes possible to better understand the complex interaction pattern of a bispecific molecule when binding to a solid support containing mixed targets. It is possible to derive an apparent affinity for the different binding modes, and also an approximate relative proportion. In this particular case, the bispecific molecule will in about 2/3 of the binding events have an apparent affinity which is 5-20 times stronger than any of the two individual interactions when binding to a mixed target solid support. In terms of characteristic value has improved a factor 20 due to the multispecific aspects of the molecule. This means that in the case this bispecific molecule was a candidate therapeutic agent, characterization with respect to the individual interactions would be misleading.

### Example 2

Also in this example, the experimental data was originally described in the report "Generation and Evaluation of Bispecific Affibody Molecules for Simultaneous Targeting of EGFR and HER2" by L Ekerljung and co-authors as published in Bioconjugate Chemistry. 2012 Sep 19;23(9):1802-11. This example relates to bispecific molecule III in Table 1 in the report by Ekerljung cited above. The time resolved binding curves for bispecific molecule III binding to (a) EGFR, (b) HER2 and (c) a mix of EGFR+HER2 were subjected to Interaction Map analysis. Figure3 shows the time resolved binding curves and the respective Interaction Map results. In more details, when processing the data from bispecific molecule III binding to a solid support where only EGFR was immobilized, the Interaction Map results (310) in a single dominant peak (311) located at position log10(ka) = 3.4 and log10(kd) = -3.2 which represents an affinity of 210 nM, which also is the characteristic value for the EGFR interaction. When processing the data from bispecific molecule III binding to a solid support where only HER2 was immobilized, the Interaction Map results (320) in a single dominant peak (321) located at position log10(ka) = 4.7 and log10(kd) = -3.5 which represents an affinity of 6.0 nM, which also is the characteristic value for the HER2 interaction. The affinity value for the EGFR interaction extracted using Interaction Map is approximately a factor three different from the values reported in Table 1 in the report by Ekerljung as cited above, due to the same reasons as discussed in Example 1.

When processing the data from bispecific molecule III binding to a solid support where a mix of EGFR and HER2 were immobilized, the Interaction Map resulted (330) in a single dominant peak (331) located at position log10(ka) = 4.6 and log10(kd) = -3.9 which represents an affinity of 2.8 nM. Hence, the characteristic value for the mixed immobilization is 2.8 nM.

Molecule III binds to a mix of EGFR and HER2 with an apparent affinity approximately 5 times stronger than the strongest individual interaction, meaning that the characteristic value has improved approximately a factor 5 due to the multispecific aspects of the molecule and further meaning that the apparent affinity has improved approximately a factor 5. The resulting binding curve behaves like a single interaction with predominantly slower dissociation rate than any of the two individual interactions, and with the association rate similar to the HER2 interaction. Hence, in practice the molecule III is first binding to HER2 followed by recruiting an EGFR for the other binding site of molecule III. The fact that molecule III anchors to the solid support through binding to both receptors, the likelihood of molecule III releasing from both at the very same time is decreased and that results in an apparent slower dissociation rate.

Although the invention has been described with regard to its preferred embodiment, which constitutes the best mode currently known to the inventor, it should be understood that various changes and modifications as would be obvious to one having ordinary skill in this art may be made without departing from the scope of the invention as set forth in the claims appended hereto.

## Claims

1. Method for the characterization of a multispecific species, said multispecific species being capable of binding at least two different defined targets, said method comprising the steps of
a) providing, for each target, at least one solid support with only one target immobilized;
b) providing at least one solid support with at least two targets immobilized;
c) contacting each solid support having only one target immobilized with a liquid containing a predefined concentration of said multispecific species and detecting in a time resolved manner the progress of the interaction of said multispecific species with the target on said solid support, so as to create at least one single-target binding curve for each target;
d) contacting each solid support having at least two targets immobilized with a liquid containing a predefined concentration of said multispecific species and detecting in a time resolved manner the progress of the interaction of said multispecific species with the targets on said solid support, so as to create at least one multi-target binding curve for each combination of targets;
e) processing each binding curve in a processor to produce a multidimensional fingerprint, said multidimensional fingerprint being a representation of the binding curve being processed;
f) in each of the multidimensional fingerprints of single-target binding curves, identifying the single dominant feature as the characteristic value for the isolated interaction of said multispecific species to the only target immobilized on the solid support;
g) in the multidimensional fingerprint of a multi-target binding curve, comparing the features of the multi-target multidimensional fingerprint to the characteristic values of the single-target multidimensional fingerprint for the targets present in said multi-target multidimensional fingerprint and calculating the greatest improvement from any of the characteristic values to the closest feature(s) in the multi-target multidimensional fingerprint as the change in binding characteristics due to multispecificity; wherein said calculating the greatest improvement is conducted as one of
I. improvement in terms of apparent binding affinity
II. improvement in terms of reduced dissociation rate
III. improvement in terms of increased association rate
IV. improvement in terms of weight of the dominant peak

2. Method according to claim 1, wherein the steps of contacting a solid support with said multispecific species comprises using two different concentrations of said multispecific species.

3. Method according to claim 1, wherein said multidimensional fingerprint is an Interaction map.

4. Method according to claim 1, wherein said characteristic value for the isolated interaction is one of the following:
a) The position of the dominant peak
b) The affinity derived from the position of the dominant peak
c) The weight of the dominant peak
d) The association rate value of the dominant peak
e) The dissociation rate value of the dominant peak
f) The width of the dominant peak

5. Method according to claim 1, wherein said binding curves are detected in an instrument based on the detection of refractive index near the surface of a solid support.

6. Method according to claim 1, wherein said binding curves are detected in an instrument having one of the following detection principles:
a) Surface Plasmon Resonance
b) Quartz Crystal Microbalance
c) Bio-Layer Interferometry
d) Surface Acoustic Wave

7. Method according to claim 1, wherein said multispecific species is labeled with either a fluorescent label or a radioactive label, and wherein said binding curves are detected using a method which relies on the temporary reduction of liquid during quantification of bound multispecific species.

8. Method according to claim 1, wherein each of said targets is a protein with molecular weight exceeding 5000 Da and wherein any of said different targets share less than 98% of the amino acid sequence with any other of said different targets.

9. Method according to claim 1, wherein said multispecific species comprises a molecule which has two distinct binding sites with different specificities.

10. Method according to claim 9, wherein said multispecific species comprises a protein molecule.

## Patentansprüche

1. Verfahren zur Charakterisierung einer multispezifischen Spezies, wobei die multispezifische Spezies in der Lage ist, mindestens zwei verschiedene definierte Targets zu binden, wobei das Verfahren die folgenden Schritte umfasst
a) Bereitstellen, für jedes Target, mindestens eines festen Trägers mit nur einem immobilisierten Target;
b) Bereitstellen mindestens eines festen Trägers mit mindestens zwei immobilisierten Targets;
c) Kontaktieren eines jeden festen Trägers, der nur ein Target aufweist, das mit einer Flüssigkeit immobilisiert ist, die eine vorbestimmte Konzentration der multispezifischen Spezies enthält, und Detektieren des Fortschritts der Interaktion der multispezifischen Spezies mit dem Target auf dem festen Träger in einer zeitaufgelösten Weise, um so mindestens eine Einzeltarget-Bindungskurve für jedes Target zu erzeugen;
d) Kontaktieren jedes festen Trägers, der mindestens zwei Targets aufweist, die mit einer Flüssigkeit immobilisiert sind, die eine vorbestimmte Konzentration der multispezifischen Spezies enthält, und Detektieren des Fortschritts der Interaktion der multispezifischen Spezies mit den Targets auf dem festen Träger in einer zeitaufgelösten Weise, um mindestens eine Multitarget-Bindungskurve für jede Kombination von Targets zu erzeugen;
e) Verarbeiten jeder Bindungskurve in einem Prozessor, um einen mehrdimensionalen Fingerabdruck zu erzeugen, wobei der mehrdimensionale Fingerabdruck eine Darstellung der verarbeitenden Bindungskurve ist;
f) Identifizieren, in jedem der mehrdimensionalen Fingerabdrücke von Einzeltarget-Bindungskurven, des einzelnen dominanten Merkmals als den charakteristischen Wert für die isolierte Interaktion der multispezifischen Spezies mit dem einzigen auf dem festen Träger immobilisierten Target;
g) Vergleichen, in dem multidimensionalen Fingerabdruck einer Multitarget-Bindungskurve, der Merkmale des Multitarget-multidimensionalen Fingerabdrucks mit den charakteristischen Werten des Einfachtargetmultidimensionalen Fingerabdrucks für die Targets, die in dem Multitarget-multidimensionalen Fingerabdruck vorhanden sind, und Berechnen der größten Verbesserung von jedem der charakteristischen Werte zu dem/den nächstliegenden Merkmal(en) in dem Multitarget-multidimensionalen Fingerabdruck als die Änderung in den Bindungscharakteristika aufgrund von Multispezifität; wobei die Berechnung der größten Verbesserung durchgeführt wird als eine von
I. Verbesserung in Bezug auf die angenommene Bindungsaffinität
II. Verbesserung in Bezug auf die reduzierte Dissoziationsrate
III. Verbesserung in Bezug auf die erhöhte Assoziationsrate
IV. Verbesserung in Bezug auf das Gewicht des dominanten Maximums

2. Verfahren nach Anspruch 1, wobei die Schritte des Kontaktierens eines festen Trägers mit der multispezifischen Spezies das Verwenden von zwei verschiedenen Konzentrationen der multispezifischen Spezies umfassen.

3. Verfahren nach Anspruch 1, wobei der mehrdimensionale Fingerabdruck eine Interaktionskarte ist.

4. Verfahren nach Anspruch 1, wobei der charakteristische Wert für die isolierte Interaktion einer der folgenden ist:
a) Die Position des dominanten Maximums
b) Die aus der Position des dominanten Maximums abgeleitete Affinität
c) Das Gewicht des dominanten Maximums
d) Der Wert der Assoziationsrate des dominanten Maximums
e) Der Wert der Dissoziationsrate des dominanten Maximums.
f) Die Breite des dominanten Maximums

5. Verfahren nach Anspruch 1, wobei die Bindungskurven in einem Instrument auf der Grundlage der Detektion des Brechungsindexes nahe der Oberfläche eines festen Trägers detektiert werden.

6. Verfahren nach Anspruch 1, wobei die Bindungskurven in einem Instrument detektiert werden, das eine der folgenden Detektionsprinzipien aufweist:
a) Oberflächenplasmonenresonanz
b) Quarzkristall-Mikrowaage
c) Bio-Schicht-Interferometrie
d) Oberflächenakustische Welle

7. Verfahren nach Anspruch 1, wobei die multispezifische Spezies entweder mit einer fluoreszierenden Markierung oder einer radioaktiven Markierung markiert wird, und wobei die Bindungskurven unter Verwendung eines Verfahrens nachgewiesen werden, das auf der temporären Reduktion von Flüssigkeit während der Quantifizierung der gebundenen multispezifischen Spezies beruht.

8. Verfahren nach Anspruch 1, wobei jedes der Targets ein Protein mit einem Molekulargewicht von mehr als 5000 Da ist und wobei jedes der verschiedenen Targets weniger als 98 % der Aminosäuresequenz mit jedem anderen der verschiedenen Targets teilt.

9. Verfahren nach Anspruch 1, wobei die multispezifische Spezies ein Molekül umfasst, das zwei ausgeprägte Bindungsstellen mit unterschiedlichen Spezifitäten aufweist.

10. Verfahren nach Anspruch 9, wobei die multispezifische Spezies ein Proteinmolekül umfasst.

## Revendications

1. Procédé de caractérisation d'une espèce multi-spécifique, ladite espèce multi-spécifique étant capable de se lier à au moins deux cibles définies différentes, ledit procédé comprenant les étapes de :
a) fournir, pour chaque cible, au moins un support solide avec une seule cible immobilisée ;
b) fournir au moins un support solide avec au moins deux cibles immobilisées ;
c) mettre en contact chaque support solide n'ayant qu'une seule cible immobilisée avec un liquide contenant une concentration prédéfinie de ladite espèce multi-spécifique et détecter avec une résolution dans le temps la progression de l'interaction de ladite espèce multi-spécifique avec la cible sur ledit support solide, de manière à créer au moins une courbe de liaison de cible unique pour chaque cible ;
d) mettre en contact chaque support solide ayant au moins deux cibles immobilisées avec un liquide contenant une concentration prédéfinie de ladite espèce multi-spécifique et détecter de manière résolue dans le temps la progression de l'interaction de ladite espèce multi-spécifique avec les cibles sur ledit support solide, de manière à créer au moins une courbe de liaison de multi-cibles pour chaque combinaison de cibles ;
e) traiter chaque courbe de liaison dans un processeur pour produire une empreinte multidimensionnelle, ladite empreinte multidimensionnelle étant une représentation de la courbe de liaison en cours de traitement ;
f) dans chacune des empreintes multidimensionnelles de courbes de liaison de cible unique, identifier la caractéristique dominante unique comme valeur caractéristique de l'interaction isolée de ladite espèce multi-spécifique avec la cible unique immobilisée sur le support solide ;
g) dans l'empreinte multidimensionnelle d'une courbe de liaison multi-cibles, comparer les caractéristiques de l'empreinte multidimensionnelle multi-cibles aux valeurs caractéristiques de l'empreinte multidimensionnelle de cible unique pour les cibles présentes dans ladite empreinte multidimensionnelle multi-cibles et calculer la plus grande amélioration de l'une quelconque des valeurs caractéristiques à la ou aux caractéristiques les plus proches dans l'empreinte multidimensionnelle multi-cibles en tant que modification des caractéristiques de liaison due à la multi-spécificité ; dans lequel ledit calcul de la plus grande amélioration est effectué comma étant l'un parmi
I. une amélioration en termes d'affinité de liaison apparente
II. une amélioration en termes de taux de dissociation réduit
III. une amélioration en termes de taux d'association augmenté
IV. une amélioration en termes de poids du pic dominant.

2. Procédé selon la revendication 1, dans lequel les étapes de mise en contact d'un support solide avec ladite espèce multi-spécifique comprennent l'utilisation de deux concentrations différentes de ladite espèce multi-spécifique.

3. Procédé selon la revendication 1, dans lequel ladite empreinte multidimensionnelle est une carte d'Interactions.

4. Procédé selon la revendication 1, dans lequel ladite valeur caractéristique de l'interaction isolée est l'une des suivantes :
a) la position du pic dominant
b) l'affinité dérivée de la position du pic dominant
c) le poids du pic dominant
d) la valeur du taux d'association du pic dominant
e) la valeur du taux de dissociation du pic dominant
f) la largeur du pic dominant.

5. Procédé selon la revendication 1, dans lequel lesdites courbes de liaison sont détectées dans un instrument basé sur la détection de l'indice de réfraction près de la surface d'un support solide.

6. Procédé selon la revendication 1, dans lequel lesdites courbes de liaison sont détectées dans un instrument présentant l'un des principes de détection suivants :
a) une résonance plasmonique de surface
b) une microbalance à cristal de quartz
c) une interférométrie à bio-couche
d) une onde acoustique de surface.

7. Procédé selon la revendication 1, dans lequel ladite espèce multi-spécifique est marquée avec un marqueur fluorescent ou un marqueur radioactif, et dans lequel lesdites courbes de liaison sont détectées en utilisant un procédé qui repose sur la réduction temporaire du liquide lors de la quantification de l'espèce multi-spécifique liée.

8. Procédé selon la revendication 1, dans lequel chacune desdites cibles est une protéine ayant un poids moléculaire supérieur à 5000 Da et dans lequel l'une quelconque desdites cibles différentes partage moins de 98% de la séquence d'acides aminés avec n'importe laquelle desdites cibles différentes.

9. Procédé selon la revendication 1, dans lequel ladite espèce multi-spécifique comprend une molécule qui possède deux sites de liaison distincts avec des spécificités différentes.

10. Procédé selon la revendication 9, dans lequel ladite espèce multi-spécifique comprend une molécule de protéine.
